# EUROPÄISCHE PATENTSCHRIFT

(11) **EP 0 809 489 B1**
(45) Veröffentlichungstag und Bekanntmachung des Hinweises auf die Patenterteilung: **16.05.2001**
(21) Anmeldenummer: 96900973.7
(22) Anmeldetag: 25.01.1996
(51) Int. Cl.: A61K 9/70, A61K 38/48, A61L 15/38

(54) **ARZNEIFORM ZUR ABGABE VON KOLLAGENASE AN WUNDEN UND VERFAHREN ZU IHRER HERSTELLUNG**
MEDICAMENT FORM FOR THE DELIVERY OF COLLAGENASE TO WOUNDS AND PROCESS FOR THE PREPARATION THEREOF
FORME GALENIQUE POUR L'ADMINISTRATION DE COLLAGENASE A DES BLESSURES ET SON PROCEDE DE PREPARATION

(30) Priorität: 02.02.1995 DE 19503338
(43) Veröffentlichungstag der Anmeldung: 03.12.1997
(73) Patentinhaber: Knoll AG, 67061 Ludwigshafen (DE)
(72) Erfinder: ROREGER, Michael, 56567 Neuwied (DE); EINIG, Heinz, 67433 Neustadt (DE)
(74) Vertreter: Flaccus, Rolf-Dieter, Dr.
(86) Internationale Anmeldenummer: EP9600294
(87) Internationale Veröffentlichungsnummer: WO9623487

(56) Entgegenhaltungen:
- EP-A- 0 049 177
- EP-A- 0 194 647
- EP-A- 0 260 645
- DE-A- 3 139 089
- DE-A- 3 606 265

## Beschreibung

In der Wundbehandlung werden wundreinigende und wundheilungsfördernde Wirkstoffe wie beispielsweise das aus dem Kulturfiltrat Clostridium histolyticum gewonnene Enzymgemisch aus verschiedenen Kollagenasen, Clostripain und neutralen Proteasen, künftig der Einfachheit halber als Kollagenase bezeichnet, die zur Entfaltung ihrer Wirkung in direkten Kontakt mit dem Wundgrund kommen müssen, mittels Arzneiformen verabreicht, die aufgrund ihrer Konsistenz auch auf sehr unebene Oberflächen lückenlos aufgebracht werden können. Zu diesen Arzneiformen zählen Lösungen, Pulver, Puder und Sprays oder halbfeste Zubereitungen wie Salben, Cremes und Gele.
Hauptnachteil dieser Arzneiform ist, daß wegen der jeweils individuellen Dosierung durch den Anwender eine exakte, reproduzierbare und gleichmäßige Dosierung von Kollagenase über die gesamte Applikationsfläche vor allem bei wiederholter Anwendung nicht möglich ist. Darüber hinaus besitzen die bekannten Arzneiformen weitere Nachteile. Lösungen, Pulver, Puder oder Sprays ermöglichen zwar eine hohe Nutzung der applizierten Kollagenase, das heißt, daß der größte Teil der verabreichten Kollagenase in der Wunde zur Wirkung gelangt, weisen aber den Nachteil auf, daß eine Steuerung der Kollagenase durch die Darreichungsform nur in sehr begrenztem Umfang möglich ist. Soll beispielsweise eine bestimmte Kollagenasekonzentration in der Wunde gleichmäßig über einen bestimmten Zeitraum aufrechterhalten werden, so ist dazu eine zeitaufwendige Behandlung notwendig mit vielen schnell freisetzenden Einzeldosen, die in relativ kurzen Intervallen appliziert werden müssen. Salben und Cremes hingegen bieten zwar deutlich mehr Möglichkeiten zur Steuerung der Kollagenasefreisetzung und Verlängerung der Wirkung, besitzen aber aufgrund ihrer Lipophilie den Nachteil der vergleichsweise geringen Ausnutzung der eingebrachten Kollagenase in der Wunde, da große Teile der Kollagenase innerhalb des Applikationszeitraums nicht an die Grenzfläche zur Wunde diffundieren und somit abgegeben werden können. Mit dem Auswaschen der Salbe oder Creme wird dieser Anteil der Kollagenase dann entfernt, ohne zur Wirkung gelangt zu sein.

Die Verwendung von modernen Wirkstoffträger- und Wirkstoffabgabesystemen wie beispielsweise Pflasterapplikationssystemen, die nicht in die Wunde eingebracht, sondern wundrandüberlappend aufgelegt werden, kommt für Kollagenase nicht in Betracht, da dieses Enzymgemisch wegen seiner proteolytischen Wirkung nur in die Wunde, nicht aber an den Wundrand oder an die die Wunde umgebende gesunde Haut gelangen darf.

Aufgabe der vorliegenden Erfindung war es daher, eine Arzneiform zu finden, welche bei einer Wundbehandlung mit Kollagenase die Nachteile konventioneller Arzneiformen vermeidet, aber deren Vorteile aufweist, und die es ermöglicht, Kollagenase auch bei wiederholter Anwendung exakt, gleichmäßig und reproduzierbar dosiert an den Wundgrund abzugeben.

Überraschenderweise wurde die Lösung in einer kollagenaseabgebenden Arzneiform gefunden, die durch eine Kombination folgender Eigenschaften gekennzeichnet ist:
a) sie besteht aus einem folienförmigen Bandmaterial
b) sie ist ausreichend flexibel und verformbar, um sich an den Wundgrund anzupassen
c) sie ist von konstanter Dicke und enthält Kollagenase in homogen verteilter Form
d) sie enthält Kollagenase in notwendiger Konzentration zur flächenspezifischen Freigabe von Kollagenase in therapeutisch an die Behandlung der Wundoberfläche angemessener Rate
e) sie ist in Flächenstücken applizierbar, die eine Überlagerung mit dem Wundrand vermeiden

Weitere zweckmäßige Ausgestaltungen der Arzneiform sind entsprechend den Merkmalen der Unteransprüche vorgesehen.

Herkömmliche verformbare, wirkstoffabgebende Arzneiformen, die nach Applikation in die Wunde flächenförmige Strukturen ausbilden, wie beispielsweise Gele, Salben, Cremes oder auch flüssige Mehrkomponentensysteme, die nach dem Zusammengeben in der Wunde unter Verfestigung miteinander reagieren, gehören nach der Fachterminologie zu den sogenannten Mehrfachdosen-Arzneiformen. Das bedeutet, daß in einem Behältnis eine Menge dieser Arzneiform enthalten ist, die für eine Vielzahl von Anwendungen mit entsprechenden Dosierungsvorgängen vorgesehen ist. Die Dosierung selbst erfolgt individuell durch den Anwender. Aussagen über die dosierte Kollagenasemenge kann der Anwender nur machen, wenn er die jeweilige Dosis vor Anwendung wiegt. Bei wiederholter Anwendung wäre die reproduzierbare Applikation einer gleichbleibenden Kollagenasemenge nur mit Hilfe eines vorgeschalteten Wägevorgangs möglich. Diese individuelle variable Dosierung ist nur möglich aufgrund der geringen Kohärenz und leichten Abteilbarkeit dieser Arzneiformen. Andererseits bietet diese geringe Kohärenz den Vorteil, daß die Arzneiform, wie erwähnt, beliebig verformt und an unebene Oberflächen angepaßt werden kann Demgegenüber ist die vorliegende erfindungsgemäße Arzneiform eine Einmaldosen-Arzneiform, die ähnlich wie Tabletten oder Kapseln kohärent und vorgeformt ist und eine definierte Kollagenasedosis für eine Anwendung in homogen verteilter Form enthält. Das hat den Vorteil, daß beliebig oft reproduzierbar die gleiche Kollagenasemenge appliziert werden kann. Unter Kohärenz wird in diesem Zusammenhang eine Festigkeit und ein innerer Zusammenhalt der Arzneiform verstanden, die im Gegensatz zu den dargestellten herkömmlichen Arzneiformen, eine Handhabung durch den Anwender ermöglicht, bei der die vorgegebene Menge der Arzneiform und damit die gegebene Kollagenasemenge durch die Handhabung selbst nicht automatisch bestimmt, verändert oder beeinflußt wird.
Die erfindungsgemäße Arzneiform unterscheidet sich von anderen Einmaldosen-Arzneiformen, wie beispielsweise Tabletten oder Kapseln, dadurch, daß sie zwar einerseits die zur Handhabung notwendige Kohärenz aufweist, andererseits aber flexibel und verformbar ist, so daß sie nach Einbringen in die Wunde an die Unebenheiten des Wundgrunds angepaßt und in Kontakt mit diesem gebracht werden kann. Vorraussetzung dafür ist, daß die flächenförmige Ausdehnung der Arzneiform kleiner oder maximal gleich der zu versorgenden Wundfläche ist. Ähnlich wie bei den genannten festen Arzneiformen wird die Homogenität der Kollagenaseverteilung dadurch erreicht, daß zunächst eine Gesamtmasse aus den Hilfsstoffkomponenten hergestellt und darin Kollagenase homogen verteilt wird. Aus einer solchen Masse wird üblicherweise im Zuge des Arzneiformungsprozesses eine Vielzahl abgeteilter Arzneiformen von gleicher Gestalt und gleichem Gewicht hergestellt, die dementsprechend alle den gleichen Kollagenasegehalt aufweisen. Typischerweise findet im Zuge der Arzneiformung durch geeignete Mittel, z. B. Ausübung von Druck oder chemische Reaktionen, eine Verfestigung statt, die der abgeteilten, einzelnen Arzneiform ihre Kohärenz verleiht.

Zur Herstellung einer erfindungsgemäßen Arzneiform wird zunächst eine niedrigviskose, fließfähige Masse, z.B. eine Lösung, eine Dispersion oder eine Schmelze, die Kollagenase in homogen verteilter Form enthält, zubereitet. Diese Masse wird dann nach dem Fachmann bekannten Verfahren auf ein flächiges Substrat beschichtet. Im Gegensatz zu festen Arzneiformen findet bei der Herstellung einer erfindungsgemäßen Arzneiform der Verfestigungsvorgang, welcher der einzelnen, abgeteilten Arzneiform ihre Kohärenz verleiht, nicht während des Arzneiformungs- und -abteilungsvorgangs statt, sondern vorher. Die Verfestigung erfolgt nach der Beschichtung auf ein flächiges Substrat durch Entzug des Lösungs- oder Dispersionsmediums mittels Trocknung bzw. durch Abkühlung, falls aus der Schmelze beschichtet wird. Der dabei stattfindende Aufbau kohäsiver Kräfte hängt in Art und Stärke von der Hilfsstoffzusammensetzung ab, worauf noch eingegangen werden wird. Es resultiert ein breites, folienförmiges Endlosband mit einer durch die Beschichtung vorgegebenen Dicke. Limitierender Faktor für die Dicke des Bandes ist bei einer gegebenen Formulierung die Forderung nach Flexibilität und Verformbarkeit der einzelnen, abgeteilten Arzneiform zur Anpassung an den Wundgrund nach Einbringen in eine Wunde. Die Abteilung einzelner Arzneiformen mit vorgegebener Fläche erfolgt aus dem Endlosband nach bekannten Verfahren wie z.B. Stanzen oder Schneiden. Da die Beschichtung mit einer Masse, die Kollagenase in homogen verteilter Form enthält, und unter Einhaltung eines konstanten Beschichtungsgewichts durchgeführt wird, enthalten alle einzeln abgeteilten Arzneiformen die gleiche Kollagenasemenge in homogener Verteilung. Dadurch wird dem Anwender eine exakte und bei wiederholter Anwendung reproduzierbare Dosierung ermöglicht.
Da Kollagenasegehalt pro Flächeneinheit und Fläche selbst durch das Herstellverfahren in breitem Rahmen stufenlos variiert werden können, bietet die erfindungsgemäße Arzneiform die Möglichkeit, auch sehr geringe Kollagenasemengen exakt und zuverlässig zu dosieren.

Darüber hinaus kann der Anwender eine an der jeweiligen Problemstellung und den Therapieerfordernissen orientierte Kollagenasedosierung vornehmen. So kann der Anwender beispielsweise mehrere Arzneiformen gleichzeitig in die Wunde einbringen und nebeneinander auf den Wundgrund applizieren. Der Anwender kann aus einer Arzneiform gegebener Fläche aber auch kleine Stücke abteilen, wenn z.B. die zu behandelnde Wundfläche kleiner als die flächenmäßige Ausdehnung der Arzneiform ist, oder wenn die durch die Fläche gegebene Kollagenasedosis der Arzneiform für eine spezielle Behandlung zu hoch ist. So kann die Arzneiform z.B. in Verbindung mit einem inerten flächenförmigen Substrat vorliegen, von dem sie sich leicht abheben läßt, wie beispielsweise einer silikonisierten Folie, und eine Einteilung im cm-Maßstab aufweisen. Da die Flächenbeladung der Arzneiform mit Kollagenase bekannt ist, kann der Anwender aus einer blattförmigen oder einer aufgerollten, bandförmigen Arzneiform die Fläche und damit die Kollagenasemenge aus- bzw. abschneiden, die er aus therapeutischer Sicht für notwendig erachtet.
In jedem Fall kann erreicht werden, daß die flächenförmige Ausdehnung der Arzneiform kleiner oder maximal gleich der zu versorgenden Wundfläche ist. Dadurch wird die Applikation an den Wundgrund ermöglicht und sichergestellt, daß die applizierte Kollagenasemenge in der Wunde freigesetzt wird. Bei wundrandüberlappender Applikation würde nur der in die Wunde reichende Teil der Arzneiform Kollagenase freisetzen, wodurch der Vorteil der exakten Dosierug zunichte gemacht würde.
Ein weiterer Vorteil der erfindungsgemäßen Arzneiform ist, daß aus ihr Kollagenase kontrolliert freigesetzt werden kann. Da die Arzneiform nach Applikation in jedem Fall mit Wund- bzw. Gewebsflüssigkeit in Kontakt tritt, hat die Interaktion mit Flüssigkeit entscheidenden Einfluß auf die Kollagenasefreisetzung, was wiederum zur Steuerung der Freisetzung genützt werden kann. So kann die Rezeptur der erfindungsgemäßen Arzneiform zur Erzielung einer relativ schnellen Kollagenasefreisetzung so ausgelegt werden, daß die Arzneiform in Wundflüssigkeit löslich bzw. zerfallbar ist. Die Freisetzungskinetik für Kollagenase hängt in diesem Fall von der Auflösungs- bzw. Zerfallsgeschwindigkeit der Arzneiform ab. Nach Ablauf der Applikationszeit muß die aufgelöste bzw. zerfallene Arzneiform, ähnlich wie Salben oder Cremes, aus der Wunde ausgewaschen werden, es sei denn, daß die Rezeptur so ausgelegt wird, daß die Vorrichtung in Wundflüssigkeit vollständig abbaubar und resorbierbar ist.
Eine Verzögerung und Verlängerung der Kollagenasefreisetzung kann erreicht werden, wenn die Zusammensetzung so gewählt wird, daß die Arzneiform unter Wundflüssigkeitsaufnahme lediglich quillt. Die Wundflüssigkeit löst insbesondere die Kollagenase aus der Arzneiform heraus, was zu deren langsamer Erosion führt. In diesem Fall hängt die Kollagenasefreisetzung vom Quellvermögen und der Erosionsgeschwindigkeit der Arzneiform ab.
Eine noch weitergehende Verzögerung und Verlängerung der Kollagenasefreisetzung wird erzielt, wenn die Zusammensetzung der Arzneiform so gewählt wird, daß sie gegenüber Wundflüssigkeit inert ist und nicht mit ihr interagiert. Die Freisetzungskinetik für Kollagenase hängt dann nur von der Diffusionsgeschwindigkeit von Kollagenase innerhalb der Arzneiform sowie von der Grenzfläche zwischen Vorrichtung und Wundgrund bzw. Wundflüssigkeit ab.
In den genannten Fällen, in denen die Arzneiform nicht löslich oder zerfallbar ist, hat der Anwender den Vorteil, daß er diese jederzeit ohne Auswaschen oder ähnliche Manipulationen vollständig aus der Wunde entfernen kann.

In einer weiteren bevorzugten Ausführungsform ist die erfindungsgemäße Arzneiform mehrschichtig aufgebaut. So kann beispielsweise eine in Wundflüssigkeit lösliche oder zerfallbare Schicht, die der schnellen Freisetzung von Kollagenase zur möglichst raschen Erreichung der mindest notwendigen Kollagenasekonzentration dient, in Laminatform zusammengebracht sein mit einer quellbaren oder einer inerten Schicht, die einer langsamen und gleichmäßigen Freisetzung von Kollagenase zur Aufrechterhaltung der notwendigen Kollagenasekonzentration über einen längeren Zeitraum dient. In einer bevorzugten Ausführungsform einer mehrschichtigen Arzneiform kann diese ein Sperr- und/oder Steuerelement aufweisen, das keine Kollagenase enthält, wie beispielsweise eine flexible Folie aus Polyurethan, Polyester oder Polypropylen. Mit Hilfe eines solchen Sperr- bzw. Steuerelements soll die Kollagenaseabgabe in eine bestimmte Richtung gelenkt werden. Wenn beispielsweise eine verformbare, kollagenaseabgebende Schicht an den Wundgrund appliziert wird, so kann durch eine darauf laminierte Sperrschicht verhindert werden, daß beispielsweise in einer stark exsudierenden Wunde Kollagenase an die umgebende Wundflüssigkeit abgegeben wird, was möglicherweise zu einem unerwünscht starken Verdünnungseffekt führen würde.
In einer weiteren bevorzugten Ausführung der erfindungsgemäßen Arzneiform für die Wundbehandlung ist die Vorrichtung porös, z.B. schaum- oder schwammartig. Die Größe der Poren und die Struktur der Arzneiform ist so ausgelegt, daß ein Einwandern von Zellen wie z.B. Fibroblasten in diese möglich ist und den Zellen dabei eine strukturelle Orientierung gegeben wird, die insbesondere auf den vorzugsweise dem natürlichen Bindegewebe ähnlichen Ordnungsgrad der Schwammstruktur in der erfindungsgemäßen Arzneiform zurückzuführen ist. Das Einwachsen der Zellen kann z.B. notwendig sein für den Abbau der Zubereitung oder zur Abgabe bzw. Ablagerung von Substanzen, die z.B. für Gewebsneubildung benötigt werden oder für die Vaskularisation eines Gewebes, das an die Stelle der erfindungsgemäßen Arzneiform nach deren Abbau treten soll. Die Vorraussetzungen für die Porosität der Arzneiform werden dabei im Zuge der Herstellung dadurch geschaffen, daß beispielsweise in die zu beschichtende Masse mit homogener Verteilung von Kollagenase Luft eingerührt wird, oder daß nach Beschichtung aus Lösung oder Dispersion durch externe Trocknungsbedingungen das verdampfende Lösemittel oder Dispersionsmedium Löcher bzw. Poren in der beschichteten Bahn hinterläßt.
Die Auswahl von Materialien und Hilfsstoffen zur Herstellung der erfindungsgemäßen Arzneiform wird zunächst bestimmt durch die Anforderungen an Kohärenz, Flexibilität und Verformbarkeit, sowie durch Anforderungen an die gewünschte Freisetzungskinetik für Kollagenase. Weiterer beschränkender Faktor ist, daß das Spektrum verwendbarer Materialien und Hilfsstoffe auf solche, die bei Kontakt mit Wundgewebe eine ausgezeichnete Verträglichkeit aufweisen, reduziert ist. Die aus einer Kombination von Materialien und Hilfsstoffen hergestellte Arzneiform soll nach Applikation in die Wunde Zellen wie beispielsweise Keratinocyten, Fibroblasten oder Endothelzellen in ihrer Funktion und Aktivität nicht behindern.
Mindestens notwendig zur Herstellung einer erfindungsgemäßen Arzneiform sind Hilfsstoffe aus der Gruppe der Polymere und Hilfsstoffe aus der Gruppe der Weichmacher. Polymere sorgen für den inneren Zusammenhalt und die Kohärenz der Arzneiform, da sie nach Beschichtung und Trocknung bzw. Abkühlung durch beispielsweise kovalente Bindungen wie Wasserstoffbrückenbindungen oder ionische Wechselbeziehungen Netzwerke ausbilden, die der Verfestigung dienen und somit die notwendige Kohärenz der Arzneiform schaffen. Durch Weichmacher wird die Konsistenz der Arzneiform so eingestellt, daß sie flexibel und verformbar und somit an den Wundgrund anpaßbar ist. Geeignete Weichmacher mit physiologischer Eignung für die Wundbehandlung sind vorzugsweise niedermolekulare, mehrwertige Alkohole wie beispielsweise Glycerin, Sorbitol, niedermolekulares Polyethylenglykol oder niedermolekulares Polypropylenglykol.
Polymere mit Eignung für eine schnellfreisetzende Arzneiform, die in Wundflüssigkeit löslich ist oder zumindest zerfällt, sind insbesondere wasserlösliche Polymere. Dazu gehören vorzugsweise pflanzliche Polysaccharide wie Alginate, Pektine, Carrageenane oder Xanthan, Cellulosederivate wie Methylcellulose, Hydroxipropylcellulose, Hydroxiethylcellulose, Hydroxipropylmethylcellulose oder Natriumcarboximethylcellulose, Stärke und Stärkederivate, Galaktomannan und Galaktomannanderivate, Chitosan und Chitosanderivate, Glykoproteine, Proteoglykane, Glucosaminoglykane, Polyvinylalkohol, Polyvinylpyrrolidon, Vinylpyrrolidon-VinylacetatMischpolymerisate, höhermolekulare Polyethylenglykole und höhermolekulare Polypropylenglykole.
Polymere mit Eignung für eine verzögert und über einen längeren Zeitraum freisetzende Arzneiform, die in Wundflüssigkeit quillt oder nicht mit Wundflüssigkeit interagiert, sind insbesondere wasserquellbare oder wasserunlösliche Polymere. Dazu gehören vorzugsweise Cellulosederivate wie Ethylcellulose, Celluloseacetatphthalat, Hydroxipropylmethylcellulosephthalat, Celluloseacetatsuccinat oder Ethylcellulosesuccinat, Polyoxiethylen-Polyoxipropylen-Copolymere, Polyvinylalkohol, Polyacrylate und Polymethacrylate, Polylactide, Polyglycolide sowie Polyaminosäuren.

Als weitere Hilfsstoffe kann die Arzneiform aufweisen:
· Konservierungsmittel, wie z.B. p-Cl-m-Kresol, Phenylethylalkohol, Phenoxiethylalkohol, Chlorbutanol, 4-Hydroxibenzoesäuremethylester, 4-Hydroxibenzoesäurepropylester, Bezalkoniumchlorid, Cetylpyridiniumchlorid, Chlorhexidindiacetat oder -digluconat, Ethanol oder Propylenglykol
· pH-Regulatoren wie z.B. Glycinpuffer, Citratpuffer, Boratpuffer, Posphatpuffer oder Citronensäure-Phosphat-Puffer
· Antioxidantien wie z.B. Ascorbinsäure, Ascorbylpalmitat, Tocopherolacetat, Propylgallat, Butylhydroxianisol oder Butylhydroxitoluol
· Hilfsstoffe zur Stabilisierung der biologischen Aktivität von Wirkstoffen wie Mannitol, Glucose, Lactose, Fructose, Saccharose, Cyclodextrin oder Dextran
· Emulgierbare Hilfsstoffe wie Öle, Fette und Wachse
· Emulsionsstabilisatoren wie z.B. nichtionogene Emulgatoren, amphotere Emulgatoren, kationaktive Emulgatoren und anionaktive Emulgatoren
· Füllstoffe wie z.B. mikrokristalline Cellulose, Aluminiumoxid, Zinkoxid, Titandioxid, Talkum, Siliciumdioxid, Magnesiumsilikat, Magnesium-Aluminiumsilikat, Kaolin, hydrophobe Stärke, Calciumstearat oder Calciumphosphat.
· Schäumungsmittel wie Saponine, Alginsäureester, Aminoxide oder Fettaminoxide

### Beispiel 1

32 g Aceton, 14,6 g Ethylacetat und 6,5 g Polyethylenglykol 400 werden in einem verschließbaren Rührgefäß vorgelegt. In dem Lösemittelgemisch werden unter gleichmäßigem Rühren nacheinander 33,6 g eines Polyvinylpyrrolidon-Polyvinylacetat-Copolymers, 2 g eines Polyoxyethylen-Polyoxypropylen-Copolymers und 9,3 g Hydroxipropylcellulose gelöst.

Danach wird in der Lösung eine Trockenmischung aus 1 g Kollagenase (kollagenolytische Aktivität 1000 U/g) und 1 g β-Cyclodextrin dispergiert. Die homogene Dispersion wird mit einem Flächengewicht von 500 g/m² auf ein silikonisiertes Papier gestrichen und konvektiv in einem Trockenkanal bei 45 °C und einer Luftgeschwindigkeit von ca. 5 m/sec getrocknet. Nach dem Trocknen wird ein weicher, flexibler, transparenter Film von bräunlicher Farbe erhalten, der ein Flächengewicht von 267 g/m² und dementsprechend einen Enzymgehalt von 0,5 mg/cm², entsprechend einer kollagenolytischen Aktivität von 0,5 Units/cm², aufweist. Aus dem Film werden rechteckige Vorrichtungen mit einer Fläche von 12 cm² und einem Enzymgehalt von 6 mg, entsprechend einer kollagenolytischen Aktivität von 6 Units, geschnitten. Die Arzneiformen werden jeweils in eine Paddle-Apparatur gegeben und in 50 ml einer 0,01 molaren Calciumacetat-Lösung mit 60 U/min gerührt. Der Lösung wird ein Überschuß eines Hexapeptids, das als Substrat für die enzymatische Spaltung durch Kollagenase dient, zugegeben. Nach 5 Minuten wird eine Probe der Lösung entnommen und versetzt mit einem Überschuß Ninhydrin, das mit den Tripeptiden, die durch die enzymatische Spaltung des Hexapeptid-Substrats entstehen, einen Farbkomplex bildet, der spektralphotometrisch vermessen wird und als Resultat eine enzymatische Aktivität von 6,36 Units ergibt.

Kollagenase ist also bereits nach 5 Minuten zu 100 % freigesetzt. Die gewünschte Anforderung an den flexiblen Film in der gewählten Zusammensetzung, nämlich eine möglichst hohe Enzymkonzentration in möglichst kurzer Zeit in der Wunde zu erzielen, kann somit erfüllt werden.

### Beipiel 2

32 g Aceton, 14,7 g Ethylacetat und 6,5 g Polyethylenglykol 400 werden in einem verschließbaren Rührgefäß vorgelegt. In dem Lösemittelgemisch werden unter gleichmäßigem Rühren nacheinander 33,1 g eines Polyvinylpyrrolidon-Polyvinylacetat-Copolymers, 1,9 g eines Polyoxyethylen-Polyoxypropylen-Copolymers und 9,3 g Hydroxipropylcellulose gelöst.

Danach wird in der Lösung eine Trockenmischung aus 1 g Kollagenase (kollagenolytische Aktivität 1000 U/g), 1 g Dextran 40 und 0,5 g Calciumacetat dispergiert. Die homogene Dispersion wird mit einem Flächengewicht von 500 g/m² auf ein silikonisiertes Papier gestrichen und konvektiv in einem Trockenkanal bei 45 °C und einer Luftgeschwindigkeit von ca. 5 m/sec getrocknet. Nach dem Trocknen wird ein weicher, flexibler, transparenter Film von bräunlicher Farbe erhalten, der ein Flächengewicht von 266,5 g/m² und dementsprechend einen Enzymgehalt von 0,5 mg/cm², entsprechend einer kollagenolytischen Aktivität von 0,5 Units/cm² aufweist. Aus dem Film werden rechteckige Arzneiformen mit einer Fläche von 12 cm² und einem Enzymgehalt von 6 mg, entsprechend einer kollagenolytischen Aktivität von 6 Units, geschnitten. Die Arzneiformen werden jeweils in eine Paddle-Apparatur gegeben und in 50 ml einer 0,01 molaren Calciumacetat-Lösung mit 60 U/min gerührt. Der Lösung wird ein Überschuß eines Hexapeptids, das als Substrat für die enzymatische Spaltung durch Kollagenase dient, zugegeben. Nach 5 Minuten wird eine Probe der Lösung entnommen und versetzt mit einem Überschuß Ninhydrin, das mit den Tripeptiden, die durch die enzymatische Spaltung des Hexapeptid-Substrats entstehen, einen Farbkomplex bildet, der spektralphotometrisch vermessen wird und als Resultat eine kollagenolytische Aktivität von 5,52 Units ergibt.

Kollagenase ist also bereits nach 5 Minuten zu mehr als 90 % freigesetzt. Die gewünschte Anforderung an den flexiblen Film in der gewählten Zusammensetzung mit Dextran und Calciumacetat zur Stabilisierung der Kollagenaseaktivität, nämlich eine möglichst hohe Kollagenasekonzentration in möglichst kurzer Zeit in der Wunde zu erzielen, kann somit erfüllt werden.

### Beispiel 3

Angestrebt wird ein flexibler Film, der im Vergleich zu den Filmen nach den Beispielen 1 und 2 eine verzögerte Freisetzung aufweist. 40 g Aceton, 20 g Ethylacetat und 7 g Polyethylenglykol 400 werden in einem verschließbaren Rührgefäß vorgelegt. In dem Lösemittelgemisch werden unter gleichmäßigem Rühren nacheinander 15 g eines Polyvinylpyrrolidon-Polyvinylacetat-Copolymers, 1,2 g eines Polyoxyethylen-Polyoxypropylen-Copolymers, 6,5 g Hydroxipropylcellulose und 8,3 g Ethylcellulose gelöst.

Danach wird in der Lösung eine Trockenmischung aus 1 g Kollagenase (kollagenolytische Aktivität 1000 U/g) und 1 g β-Cyclodextrin dispergiert. Die homogene Dispersion wird mit einem Flächengewicht von 500 g/m² auf ein silikonisiertes Papier gestrichen und konvektiv in einem Trockenkanal bei 45 °C und einer Luftgeschwindigkeit von ca. 5 m/sec getrocknet. Nach dem Trocknen wird ein weicher, flexibler, transparenter Film von bräunlicher Farbe erhalten, der ein Flächengewicht von 200 g/m² und dementsprechend einen Enzymgehalt von 0,5 mg/cm², entsprechend einer kollagenolytischen Aktivität von 0,5 Units/cm², aufweist. Aus dem Film werden rechteckige Arzneiformen mit einer Fläche von 12 cm² und einem Kollagenasegehalt von 6 mg, entsprechend einer kollagenolytischen Aktivität von 6 Units, geschnitten. Die Vorrichtungen werden jeweils in eine Paddle-Apparatur gegeben und in 50 ml einer 0,01 molaren Calciumacetat-Lösung mit 60 U/min gerührt. Der Lösung wird ein Überschuß eines Hexapeptids, das als Substrat für die enzymatische Spaltung durch Kollagenase dient, zugegeben. Nach 5 und 60 Minuten wird eine Probe der Lösung entnommen und versetzt mit einem Überschuß Ninhydrin, das mit den Tripeptiden, die durch die enzymatische Spaltung des Hexapeptid-Substrats entstehen, einen Farbkomplex bildet, der spektralphotometrisch vermessen wird und als Resultat nach 30 Minuten eine enzymatische Aktivität von 5,64 Units ergibt.
Kollagenase ist nach 5 Minuten in deutlich geringerem Maß als in den Beispielen 1 bzw. 2 und erst nach 60 Minuten zu mehr als 90 % freigesetzt.
Die gewünschte Anforderung an den flexiblen Film in der Zusammensetzung mit Zugabe von Ethylcellulose, nämlich eine vergleichsweise verzögerte Freisetzung von Kollagenase über mindestens 60 Minuten hinweg zu erzielen, kann somit erfüllt werden.

### Beispiel 4:

Angestrebt wird ein flexibler Film, der im Gegensatz zu den drei vorher genannten Beispielen ohne Verwendung organischer Lösemittel hergestellt wird und der eine sehr schnelle Freisetzung aufweist.

67,75 Polyethylenglykol 1500 werden zusammen mit 6,0 g Polyethylenglykol 400 bei einer Temperatur von 90 °C in einem Glasgefäß unter Rühren geschmolzen. In der Schmelze werden 20,0 g eines Polyvinylpyrrolidon/Vinylacetat-Copolymeren bei 90 °C unter Rühren gelöst. Nach Abkühlen der Schmelze auf 45 °C werden 6,25 % eines Gemisches aus Kollagenase, Dextran 40 und Calciumacetat (Masseverhältnis 2:2:1, Aktivität der Kollagenase 1000 U/g) portionsweise zur Masse gegeben und durch Rühren dispergiert. Die homogene Masse wird anschließend auf PETP-Folie zu einem Film mit dem Flächengewicht von 400 g/m² ausgestrichen. Nach dem Erkalten erhält man einen weichen, flexiblen und weitgehend transparenten Film von bräunlicher Farbe, der eine kollagenolytische Aktivität von 1 U/cm² aufweist. Nach Abdecken mit einer zweiten PETP-Folie werden aus diesem Film quadratische Arzneiformen mit abgerundeten Ecken und Flächen von 25 cm², entsprechend 25 U Kollagenase, ausgestanzt.

Die Arzneiformen lösen sich in physiologischer Kochsalzlösung innerhalb von 5 Minuten vollständig auf und geben dabei den Wirkstoff Kollagenase zu 100 % frei. Sie erfüllen damit die Anforderung, innerhalb sehr kurzer Zeit große Mengen des Wirkstoffs am Applikationsort freizusetzen. Darüber hinaus entfällt durch das Herstellungsverfahren das Problem des Heraustrocknens von Lösemittelresten aus dem Film.

### Beispiel 5

Angestrebt wird ein Film, der analog zu Beispiel 4 ohne Verwendung organischer Lösemittel hergestellt wird, der jedoch eine stark verzögerte Freisetzung aufweist und in wässrigen Medien unlöslich ist.

27,0 g Vaseline werden bei 120 °C mit 15,0 g mittelkettigen Triglyceriden aufgeschmolzen. In der Schmelze werden nacheinander unter Rühren 20,0 g Poloxamer, 10,0 g Cetylstearylalkohol und 20,0 g eines Ethyl-/Vinylacetat-Copolymers geschmolzen bzw. gelöst. Die klare und homogene Masse wird unter Rühren abgekühlt auf 45 °C. Zur abgekühlten Masse werden anschließend nacheinander 1,75 g eines Gemisches aus Dextran 40 und Calciumacetat (Masseverhältnis 2:1) sowie 6,25 g eines Gemisches aus Kollagenase, Dextran 40 und Calciumacetat (Masserverhältnis 2:2:1, Aktivität der Kollagenase 1000 U/g) hinzugegeben. Der Ansatz wird bis zum Erreichen einer makroskopisch homogenen Verteilung gerührt. Die Masse wird anschließend auf PETP-Folie zu einem Film mit dem Flächengewicht von 400 g/m² ausgestrichen. Nach dem Erkalten erhält man einen weichen, sehr flexiblen, leicht opaquen Film von bräunlicher Farbe, der eine kollagenolytische Aktivität von 1 U/cm² aufweist. Nach Abdecken mit einer zweiten PETP-Folie werden aus diesem Film quadratische Arzneiformen mit abgerundeten Ecken und Flächen von 25 cm², entsprechend 25 U Kollagenase ausgestanzt.

Die Arzneiformen sind unlöslich in physiologischer Kochsalzlösung. Sie setzen den Wirkstoff Kollagenase kontinuierlich über einen Zeitraum von 24 h frei, wobei sie sich allmählich entfärben. Nach vollständiger Wirkstoffabgabe lassen sich die inzwischen weißen Arzneiformen im Ganzen wieder aus der Prüflösung entnehmen. Sie erfüllen damit die Anforderungen (1), über einen langen Zeitraum kontinuierlich Wirkstoff abzugeben und (2) jederzeit, ohne Auswaschen oder ähnliche Manipulationen, vom Applikationsort entfernbar zu sein.

## Patentansprüche

1. Arzneiform zur Abgabe von Kollagenase an Wunden, gebildet durch ein Kollagenase kontrolliert freigebendes folienförmiges Bahnmaterial von ausreichender Flexibilität und Verformbarkeit zur Anpassung an den Wundgrund, mit konstanter Dicke und homogener Verteilung von Kollagenase in notwendiger Konzentration zur flächenspezifischen Freigabe von Kollagenase in therapeutisch an die Behandlung der Wundoberfläche angemessener Rate, wobei das Bahnmaterial in Flächenstücken applizierbar ist, die eine Überlappung der Wundränder vermeiden.

2. Arzneiform nach Anspruch 1, dadurch gekennzeichnet, daß sie mehrteilig und in mehreren kleinen Teilen in eine Wunde einbringbar ist.

3. Arzneiform nach Anspruch 1, dadurch gekennzeichnet, daß sie einteilig und vor Applikation zum Einbringen in eine Wunde individuell auf die Fläche der Wunde zuschneidbar ist.

4. Arzneiform nach einem oder mehreren der Ansprüche 1 bis 3, dadurch gekennzeichnet, daß sie in Wundflüssigkeit löslich bzw. zerfallbar ist, wobei die Freisetzungskinetik für Kollagenase von der Auflösungs- bzw. Zerfallsgeschwindigkeit der Arzneiform abhängt.

5. Arzneiform nach einem oder mehreren der Ansprüche 1 bis 4, dadurch gekennzeichnet, daß sie in Wundflüssigkeit abbaubar und resorbierbar ist.

6. Arzneiform nach einem oder mehreren der Ansprüche 1 bis 3, dadurch gekennzeichnet, daß sie in Wundflüssigkeit quellbar ist, wobei die Freisetzungskinetik für Kollagenase von ihrer Erosionsgeschwindigkeit abhängt.

7. Arzneiform nach einem oder mehreren der Ansprüche 1 bis 3, dadurch gekennzeichnet, daß sie gegenüber der Wundflüssigkeit inert ist, und die Freisetzungskinetik für Kollagenase nur von der Diffusionsgeschwindigkeit von Kollagenase innerhalb der Arzneiform sowie von der Grenzfläche zwischen Vorrichtung und Applikationsstelle bzw. Wundflüssigkeit abhängt.

8. Arzneiform nach einem oder mehreren der Ansprüche 1 bis 7, dadurch gekennzeichnet, daß sie einen mehrschichtigen Aufbau aufweist.

9. Arzneiform nach einem oder mehreren der Ansprüche 1 bis 8, dadurch gekennzeichnet, daß sie zur Lenkung der Kollagenaseabgabe in eine bestimmte Richtung mindestens ein Sperr- und/oder Steuerelement aufweist.

10. Arzneiform nach einem oder mehreren der Ansprüche 1 bis 9, dadurch gekennzeichnet, daß sie mindestens einen Hilfsstoff aus der Gruppe der die Freisetzungsgeschwindigkeit beeinflussenden Polymere und Hilfsstoffe aus der Gruppe der Weichmacher enthält.

11. Arzneiform nach Anspruch 10, dadurch gekennzeichnet, daß sie wasserlösliche Polymere enthält.

12. Arzneiform nach Anspruch 10, dadurch gekennzeichnet, daß sie wasserquellbare und/oder wasserunlösliche Polymere enthält.

13. Verfahren zur Herstellung einer Arzneiform zur Abgabe von Kollagenase an Wunden, dadurch gekennzeichnet, daß zunächst eine niedrigviskose, fließfähige Masse, eine Lösung, eine Dispersion oder eine Schmelze, die Kollagenase in homogen verteilter Form enthält, zubereitet und auf ein flächiges Substrat beschichtet wird, wonach eine Verfestigung der Masse durch Entzug des Lösungs-oder Dispersionsmediums mittels Trocknung oder bei Schmelze durch Abkühlung vorgenommen wird, wobei ein folienförmiges Flächenmaterial mit einer durch die Beschichtung vorgegebenen Dicke resultiert, woraus zuletzt durch Stanzen oder Schneiden eine Anzahl von Arzneiformen von gleicher Gestalt und gleichem Gewicht abgeteilt werden.

## Claims

1. Administration form for the delivery of collagenase to wounds, formed by a film-like sheet material releasing collagenase in controlled manner and being of sufficient flexibility and deformability for adaptation to the wound surface, with constant thickness and homogeneously distributed collagenase in a necessary concentration for area-specific release of collagenase at a suitable rate for therapeutic treatment of the wound surface, the said sheet material being applicable in superficial extensions which avoid an overlap with the wound lips.

2. Administration form according to claim 1, characterised in that it consists of a plurality of parts and can be introduced into a wound in a plurality of small pieces.

3. Administration form according to claim 1, characterised in that it is undivided and, in order to introduce it into a wound, can individually be cut to the area of the wound prior to application.

4. Administration form according to one or several of the claims 1 to 3, characterised in that it is soluble or decomposable in wound fluid, the release kinetics for collagenase being dependent on the dissolution or decomposition rate of the administration form.

5. Administration form according to one or several of the claims 1 to 4, characterised in that it is degradable and absorbable in wound fluid.

6. Administration form according to one or several of the claims 1 to 3, characterised in that it is swellable in wound fluid, the release kinetics for collagenase being dependent on its erosion rate.

7. Administration form according to one or several of the claims 1 to 3, characterised in that it is inert to the wound exudate and that the release kinetics for collagenase only depends on the diffusion rate of collagenase within the administration form and on the interface between the device and application site or wound fluid.

8. Administration form according to one or more of the claims 1 to 7, characterised in that it has a multilayered structure.

9. Administration form according to one or more of the claims 1 to 8, characterised in that it has at least one barrier and/or control element to guide the collagenase release into a certain direction.

10. Administration form according to one or more of the claims 1 to 9, characterised in that it contains at least one auxiliary agent from the group of polymers influencing the release rate and auxiliary agents from the group of plasticizers.

11. Administration form according to claim 10, characterised in that it contains water-soluble polymers.

12. Administration form according to claim 10, characterised in that it contains water-swellable and/or water-insoluble polymers.

13. Process for the production of an administration form for the delivery of collagenase to wounds, characterised in that a low-viscous, flowable mass, a solution, a dispersion, or a melt, which contain collagenase in homogeneously distributed form, is prepared first and then coated on a sheet-like substrate, whereupon a solidification of the mass is effected by removal of the solvent or dispersion medium by means of drying or, in case of a melt, by means of cooling, resulting in a film-like sheet material having a thickness predetermined by the coating from which a number of administration forms of the same shape and same weight are separated at last, by punching or cutting.

## Revendications

1. Forme galénique pour l'administration de collagénase à des blessures, formée d'un matériau en bande sous forme d'un film libérant de manière contrôlée de la collagénase, d'une flexibilité et d'une déformabilité suffisantes pour l'adaptation au fond de la blessure, d'épaisseur constante et à distribution homogène de la collagénase en concentration requise pour la libération par unité de surface de collagénase à un taux thérapeutique approprié au traitement de la surface de la blessure, le matériau en bande étant applicable par parties planes qui évitent un recouvrement des bords de la blessure.

2. Forme galénique selon la revendication 1, caractérisée en ce qu'elle est constituée de plusieurs parties et en ce qu'elle peut être introduite dans une blessure en plusieurs petites parties.

3. Forme galénique selon la revendication 1, caractérisée en ce qu'elle est constituée d'une seule partie et en ce qu'elle peut être découpée individuellement d'après la surface de la blessure avant l'application en vue de son introduction dans une blessure.

4. Forme galénique selon une ou plusieurs des revendications 1 à 3, caractérisée en ce qu'elle est soluble, respectivement décomposable dans l'exsudat de la blessure, la cinétique de libération de la collagénase étant fonction de la vitesse de dissolution, respectivement de décomposition de la forme galénique.

5. Forme galénique selon une ou plusieurs des revendications 1 à 4, caractérisée en ce qu'elle est dégradable et résorbable dans l'exsudat de la blessure.

6. Forme galénique selon une ou plusieurs des revendications 1 à 3, caractérisée en ce qu'elle est gonflable dans l'exsudat de la blessure, la cinétique de libération de la collagénase étant fonction de sa vitesse d'érosion.

7. Forme galénique selon une ou plusieurs des revendications 1 à 3, caractérisée en ce qu'elle est inerte vis-à-vis de l'exsudat de la blessure et en ce que la cinétique de libération de la collagénase n'est fonction que de la vitesse de diffusion de la collagénase à l'intérieur de la forme galénique, de même que de l'interface entre le dispositif et l'endroit d'application, respectivement l'exsudat de la blessure.

8. Forme galénique selon une ou plusieurs des revendications 1 à 7, caractérisée en ce qu'elle présente une structure multicouche.

9. Forme galénique selon une ou plusieurs des revendications 1 à 8, caractérisée en ce qu'elle présente au moins un élément de barrage et/ou de contrôle pour l'orientation de la libération de collagénase dans une direction déterminée.

10. Forme galénique selon une ou plusieurs des revendications 1 à 9, caractérisée en ce qu'elle contient au moins un adjuvant du groupe des polymères influençant la vitesse de libération et des adjuvants du groupe des plastifiants.

11. Forme galénique selon la revendication 10, caractérisée en ce qu'elle contient des polymères solubles dans l'eau.

12. Forme galénique selon la revendication 10, caractérisée en ce qu'elle contient des polymères gonflables dans l'eau et/ou insolubles dans l'eau.

13. Procédé de préparation d'une forme galénique pour l'administration de collagénase à des blessures, caractérisé en ce qu'on prépare d'abord une masse fluide de viscosité réduite, une solution, une dispersion ou une masse en fusion, qui contient de la collagénase distribuée de manière homogène, et en ce qu'on l'étend sur un substrat plan, en ce qu'on procède ensuite à la solidification de la masse par retrait du milieu de mise en solution ou de mise en dispersion par séchage ou, dans le cas d'une masse en fusion, par refroidissement, donnant un matériau plan sous forme d'un film d'une épaisseur prédéterminée par le revêtement et en ce qu'on le divise finalement par poinçonnage ou par découpe en un nombre de formes galéniques de même forme et de même poids.
